# EUROPEAN PATENT APPLICATION

(11) **EP 4 751 649 A1**
(43) Date of publication of application: **03.06.2026**
(21) Application number: 24216438.2
(22) Date of filing: 29.11.2024
(51) Int. Cl.: A61B 6/03, A61B 5/11, A61B 6/00, G06T 7/254

(54) **CAMERA-BASED SUBJECT MOTION MONITORING**

(71) Applicant: Koninklijke Philips N.V., 5656 AE Eindhoven (NL)
(72) Inventor: WEISS, Steffen, Eindhoven (NL)
(74) Representative: Philips Intellectual Property & Standards

(57) **Abstract**

A medical imaging system comprising an imaging device configured to acquire a medical image of a subject and a system for monitoring motion of a subject during medical imaging procedure are provided, as well as a method for monitoring motion of a subject and a computer program product to carry out the method. The medical imaging system comprises a camera configured to acquire image frames of the subject, wherein the image frames are acquired at a camera acquisition frequency. The medical imaging system also comprises a light source configured to illuminate the subject with a modulated frequency, wherein the modulation frequency is synchronized with the camera acquisition frequency. The system further comprises a motion detection unit configured to determine a subject motion by applying a motion detection algorithm to the acquired image frames.

## Description

### FIELD OF THE INVENTION

The invention generally relates to camera-based subject motion monitoring in medical imaging. Disclosed are a subject motion monitoring system and method, a medical imaging system, and a computer program product.

### BACKGROUND OF THE INVENTION

In medical imaging devices such as computed tomography (CT) and magnetic resonance imaging (MRI) motion of a subject undergoing a medical imaging procedure, for example a medical imaging scan, can have severe impact on quality of acquired medical images. The quality of the acquired images may be affected by a wide range of subject motion. For example, the subject motion may be large voluntary movement of body parts such as movement of arms, legs, head or any other body part of the subject, or it may be small involuntary motion such as respiration induced motion or even cardiac motion. Such voluntary or involuntary motion of the subject leads to substandard image quality resulting in erroneous diagnosis or in some cases would require retaking whole or a part of the imaging scan. This not only increases the time and cost of the scan but also results in increased subject stress and dissatisfaction.

To monitor the subject motion cameras are installed within and/or outside a bore of such medical imaging devices. The cameras monitor the subject and derive a measure of the subject motion to indicate a scan image that is impaired by subject motion. In case of CT imaging, the motion signal derived from the camera may be used to shut down a tube of the CT system to avoid unnecessary radiation dose to the subject or the motion information may be used to improve image reconstruction from the acquired image data.

As mentioned above, the image quality may be affected by a wide range of subject motion, and for robust motion detection it is desired that the camera is able to monitor even minor subject motion such as respiration induced motion or cardiac motion during any medical imaging procedure.

Therefore, there is need for improved subject motion monitoring during medical imaging procedures with enhanced motion detection capabilities to increase the quality of medical image acquisition and reconstruction and/or avoiding unnecessary radiation dose to the subject.

### SUMMARY OF THE INVENTION

The current invention seeks to provide an approach for monitoring subject motion using a camera-based system with high sensitivity to subject motion. The current invention, additionally or alternatively, seeks to provide an approach for monitoring subject motion such that the motion detection is not perturbed by external visual or optical noise or disturbances. The invention has the advantage that it provides robust motion monitoring of a subject on a movable subject table which is configured to support the subject during medical imaging procedures. The current invention additionally seeks to provide an approach to continuously monitor subject motion during medical imaging procedures.

Thereto a medical imaging system comprising an imaging device to acquire medical image of a subject and a subject motion monitoring system are provided, as well as a method for monitoring subject motion during medical imaging procedures and a computer program product comprising instructions for causing a processor to carry out the method.

The medical imaging system comprises an imaging device, which imaging device is configured to acquire a medical image of a subject; a camera configured to acquire image frames of the subject, wherein the image frames are acquired at a camera acquisition frequency; a light source configured to illuminate the subject with a modulated frequency, wherein the modulation frequency is synchronized with the camera acquisition frequency; a motion detection unit configured to determine a subject motion by applying a motion detection algorithm to the acquired image frames, wherein the motion detection algorithm is applied to a difference image of two acquired image frames. Thus, the medical imaging system has the advantage that any noise caused by external disturbances that may impact the accuracy or sensitivity of subject motion detection is taken into account and compensated.

In an embodiment the frequency of the light source is modulated by switching the light source on and off. This provides an advantage of cancellation of the noise in the difference image and a more robust motion monitoring.

In another embodiment, the camera is configured to operate in a phase locked mode with the illumination of the light source. In further embodiment the camera acquires subsequent image frames with and without the subject illumination. This embodiment enables the acquisition of frames by the camera to be precisely triggered and synchronized with the illumination of the subject by the light source.

In an option, the light source is configured to illuminate the subject with an intensity that varies within a certain limit in at least one spatial direction. With the subject moving on the table with a given speed in this direction this translates into a limited variation of the brightness of any region on the subject in time. This limited variation in time simplifies the motion detection, e.g., using an optical flow algorithm. This algorithm assumes that intensity variations of regions on the subject are small, and rather regions are flowing across the image with almost constant brightness. This configuration of the light source ensures that any assumption of almost constant subject illumination by motion detection algorithms is not violated.

According to an embodiment, the imaging device is configured to receive ambient light illumination from at least one ambient light source. In this way the imaging device can be placed anywhere in the examination room without any restrictions on the ambient lighting conditions that would have an effect on the motion detection algorithm. This provides advantage that any artefact occurring in the motion detection algorithm due to shadows or other spatial variations caused by the ambient light source on the subject, or due to movement of this shadow across the subject during moving table imaging procedures will be minimized or cancelled. Furthermore, any changes in brightness or color of any observed body part of the subject will not affect motion monitoring. Thus, ambient lighting will not impair subject motion monitoring.

According to one aspect, at least a frequency of the light source and the camera acquisition frequency are configured to be synchronized with a frequency of the ambient light source. An advantage of this approach is that the motion detection algorithm will not be disturbed by any intensity fluctuation or switching of the ambient light source.

According to another aspect, the camera may be configured to detect a temporal mean ambient light level, wherein the temporal mean ambient light level is substantially equal for any two subsequent image frames acquired by the camera. This has an advantage that in the event of any fluctuation in the ambient light source and more specifically fluctuation at a frequency near the camera acquisition frequency, the motion detection algorithm remains unperturbed resulting in a robust motion monitoring system. Thus, any fluctuation in the ambient lighting will not impair subject motion monitoring.

According to still another aspect, the imaging device comprises a movable subject table which is configured to support the subject during the imaging procedure. This provides an advantage that the motion monitoring system is capable of monitoring subject motion even in presence subject table movement during a medical imaging procedure. Furthermore, as mentioned above any artefact due to shadow or spotlights cast on the subject by the ambient lighting or movement of that shadow or spotlights across the subject due to subject table movement does not impact motion detection.

In another embodiment, the motion detection unit and the camera are built as a single unit. This makes the motion monitoring system compact and easy to install anywhere outside or inside the bore of the imaging device.

In an embodiment, the imaging device is a computed tomography imaging system or a magnetic resonance imaging system. Thus, the invention has advantages in that the subject motion detection and compensation can be accomplished with high sensitivity in such devices, thereby leading to high quality medical image acquisition and reconstruction.

In a further embodiment, light exposure time of the acquired camera frames is below a predetermined threshold. This prevents saturation of camera sensors and allows for motion monitoring for a wide range of ambient lighting conditions.

In another aspect, a system for monitoring motion of a subject during a medical imaging procedure is provided. The system comprises a camera configured to acquire image frames of the subject, wherein the frames are acquired at a camera acquisition frequency; a light source configured to illuminate the subject with a modulated frequency, wherein the modulation frequency is synchronized with the camera acquisition frequency; a motion detection unit configured to determine a subject motion by applying a motion detection algorithm to the acquired image frames, wherein the motion detection algorithm is applied to a difference image of two acquired image frames. Thus, the subject motion monitoring system monitors subject motion in the presence of any external visual or optical disturbances or noise and is configured to be independently mounted on an imaging device such that the imaging device may acquire and reconstruct images with improved image quality in the presence of any subject motion.

According to a further aspect, a method for monitoring motion of a subject during a medical imaging procedure is provided. The method comprises acquiring image frames of the subject by a camera, wherein the frames are acquired at a camera acquisition frequency; illuminating the subject with light with a modulated frequency, wherein the modulation frequency is synchronized with the camera acquisition frequency, determining the subject motion by applying a motion detection algorithm to the acquired image frames, wherein the motion detection algorithm is applied to a difference image of two acquired image frames.

Additionally, a computer program product comprising instructions for causing a processor to carry out the above-described method when the computer program is executed is also provided.

An advantage of the present invention is a more robust and accurate motion monitoring in the presence of ambient noise or disturbances, for example in the presence of any shadows or spotlights cast on the subject by one or more ambient lights. Such shadows or spotlight often goes unnoticed or undetected and adversely affects the motion detection. Another advantage of the invention is that subject motion can be accurately monitored in the presence of a moving subject table. Still another advantage of the invention is that the subject motion can be accurately monitored in the presence of a moving subject table during medical image acquisitions. Thus, any shadow or spotlight that is cast on the subject and which also moves along with the subject table will not affect the motion detection algorithm. The invention additionally provides an advantage that motion monitoring can be accomplished irrespective of different subject table speeds.

The invention additionally or alternatively provides the advantage that subject motion monitoring is accomplished without any restriction to the type of ambient light source. The subject motion monitoring can be performed with different types of ambient light sources and modulation of the light output of such sources at different frequencies. This provides a cost-effective implementation of the invention without requiring any changes to the ambient light source or any additional hardware or electronics. Moreover, the invention allows for placement of the medical imaging system in the examination room without any restriction to its position with respect to the ambient light output.

### BRIEF DESCRIPTION OF THE FIGURES

In the following drawings:
Fig. 1 schematically and exemplarity illustrates a medical imaging system for acquiring medical image data of a subject comprising an imaging device and a motion monitoring system.
Figs 2a and 2b are illustrative camera images of a subject with a shadow of a CT gantry cast on the subject for different subject table positions.
Fig. 3 is an illustrative image of a subject on a CT table.
Fig. 4 is an image showing motion data of a subject.
Fig. 5 is a flow chart illustrating a method for monitoring subject motion.
Fig. 6 illustrates graphically synchronization of camera frames with the subject lighting and ambient illumination.

### DETAILED DESCRIPTION OF THE INVENTION

In the examples below, medical image data is acquired for a subject. Such a subject can be a human being, particularly a human patient requiring imaging for medical purposes. However, alternative subjects are also envisaged, for example animals such as research animals, pets, or livestock. In the examples, the imaging device is illustrated as a computed tomography (CT) scanner, but other devices may also be used to acquire the medical image data.

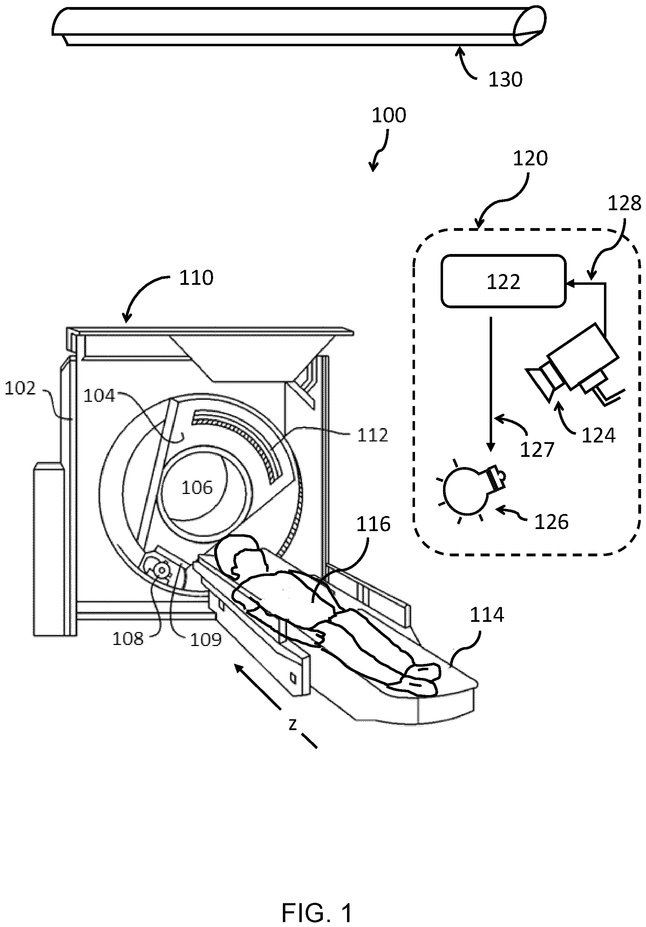

Fig. 1 shows a medical imaging system 100 for acquiring medical image of a subject.

The medical imaging system 100 comprises an imaging device 110 that is configured to acquire a medical image of the subject. The imaging device in this exemplary embodiment is a computed tomography scanner. In another embodiment imaging device may be a magnetic resonance imaging system. The computed tomography imaging device 110 includes a stationary gantry 102 and may also include a rotating gantry 104, which is rotatably supported by the stationary gantry 102. When the system is in operation, the rotating gantry 104 rotates around examination region 106 about a longitudinal or z-axis. A radiation source 108, such as an x-ray tube, is supported by and rotates with the rotating gantry 104 and emits radiation that traverses the examination region 106. A source collimator 109 collimates the emitted radiation to form a generally fan, wedge, or cone shaped radiation that traverses the examination region 106.

A subject table 114, such as a bed or couch, is provided to support an object or subject to be imaged in the examination region 106. Subject table 114 is movable along the z-axis in coordination with the rotation of the rotating gantry 104 to facilitate the desired scanning trajectory, preferably a helical scanning trajectory.

A radiation sensitive detector array 112 detects radiation emitted by the radiation source 108 that has traversed the examination region 106 and generates projection image data indicative of the detected radiation. The illustrated radiation sensitive detector array 112 includes one or more rows of radiation sensitive photosensor pixels along the z-axis.

In the medical imaging system 100, a subject motion monitoring system 120 is provided. The subject motion monitoring system 120 comprises a camera 124, a light source 126 and a motion detection unit 122.

The system 120 for monitoring motion can be integrated into the imaging device 110 but can be a separate module that may be configured so that it can be attached to or used with the imaging device 110.

The camera 124 is a motion monitoring camera capable of monitoring motion or movement of any object or subject in its field-of-view. The camera monitors motion by acquiring one or more image frames of the field-of-view. In an embodiment the camera monitors motion by comparing the one or more acquired image frames using a motion detection unit 122. In an example the image frames may be compared by determining any changes to the pixels of any two acquired camera frames. The image frames are acquired at a camera acquisition frequency. The camera acquisition frequency is selected such that the camera is able to capture even minor motions of the subject in sufficient detail such as motion due to respiration or cardiac motion. The camera 124 may be positioned or attached to an outside or an inside of a bore of the imaging device 110. In another embodiment, the camera 124 is positioned at any location outside the imaging device 110, for example it may be suspended from a ceiling of an examination room where the imaging device 110 is located.

The light source 126 may be any light source that is capable of illuminating the subject at least partially or fully from at least one spatial direction. For example, the light source 126 may be a light-emitting-diode (LED), an incandescent light source, a fluorescent light source, a discharge lamp or a tungsten light source. The light source may be a broadband or a monochromatic light source. Other than the aforementioned examples, other types of light sources may be envisaged without deviating from the scope of the invention. The light source 126 is configured to illuminate the subject with a modulated frequency. The modulation frequency of the light source is synchronized with the camera acquisition frequency. The frequency of the light source 126 may be modulated independently without the light source being connected to any other device. In an alternate embodiment the frequency of the light source 126 may be modulated by connecting the light source to a separate processor or to the motion detection unit 122 which may be configured to control the modulation and/or the operation of the light source 126 through a wired or wireless communication link 127. In this embodiment the processor or the motion detection unit 122 will synchronize the frequency of the light source with the camera acquisition frequency. In an embodiment of the invention the light source 126 may be further configured to illuminate the subject with an intensity that has a limited spatial variation in at least one spatial direction coinciding with the table movement direction. This is advantageous since with the subject moving on the table with a given speed in this direction this translates into a limited variation of the brightness of any region on the subject in time. This limited variation in time simplifies motion detection, e.g., using an optical flow algorithm. This algorithm assumes that intensity variations of regions on the subject are small, and rather regions are flowing across the image with almost constant brightness. This configuration of the light source ensures that any assumption of an almost constant subject illumination by motion detection algorithms is not violated.

The subject motion monitoring may be disturbed by external or ambient noise, in particular optical and/or visual noise. In an example it may be shadows or spotlights cast on the subject by the imaging device, equipment or even the presence of staff near the subject. Such shadows or spotlight may not even be static and may move spatially with time.

According to the invention, a motion detection unit 122 is provided. The camera 124 is connected to the motion detection unit 122 through a communication link 128. The communication link 128 may be a wired or wireless communication link. Alternatively, the motion detection unit 122 is in-built or integrated with the camera. With this alternative no separate motion detection unit may be required, and the camera is able to monitor subject motion as a standalone device. The motion detection unit may be implemented using one or more processors. The motion detection may be accomplished using a motion detection algorithm, for example an optical flow algorithm. Any other type of motion detection algorithm may be envisaged without deviating from the scope of the invention. The motion detection unit applies motion detection algorithm to the acquired image frames. The motion detection algorithm is applied to a difference image of two acquired image frames. The difference image is determined by subtracting two images, in an example by the motion detection unit. The difference image may be determined using the processor of the motion detection unit or the motion detection algorithm. Thus, by applying motion detection algorithm to the difference image any effect of external or ambient noise on the motion detection is eliminated and a robust motion detection is realized. In an embodiment, the motion detection algorithm is configured to determine subject motion by comparing one or more difference images and determining changes to the pixels of two difference images.

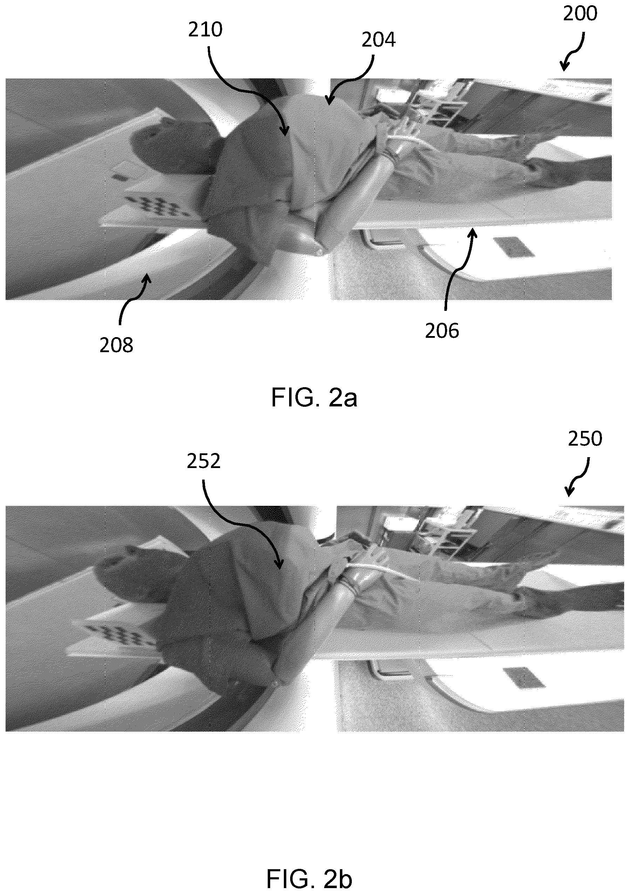

As illustrated in Fig. 1, and in an embodiment of the invention, the imaging device 110 is configured such that it can be illuminated by an ambient light source 130. The ambient light source 130 may be any type of light source, for example a light-emitting-diode (LED), an incandescent light source, a fluorescent light source, a discharge lamp, a tungsten light source or even multiple light sources of abovementioned types. In an alternate embodiment the ambient light source may be a natural light source. The imaging device 110 is configured such that it receives illumination from the ambient light source 130 so that an operator of the imaging device is able to visualize the imaging device 110 and/or objects placed inside the examination room. Thus, the ambient light source 130 assists an operator of the imaging device 110 in performing any operations on the imaging device by providing sufficient light illumination in the examination room. According to an exemplary embodiment of the invention, any illumination by ambient lights may disturb the motion detection due to the shadows or spotlights cast on the subject by the ambient lights. This will be further described in more detail with respect to Fig. 2.

With reference to Fig. 1 and in another exemplary embodiment of the invention, the frequency of the light source 126 is modulated for example by switching the light source on and off. Preferably, the rate of this modulation is beyond the temporal resolution of the human eye so that the subject and staff in the examination room are not disturbed by the flickering of the light source. As described above, since the frequency of the light source is synchronized with the camera acquisition frequency, the camera may be configured to acquire subsequent image frames with and without illumination by the light source. In an example, if the light source is pulsed at a rate f_{b}, for example 50Hz, then the light is on for 10ms and off for 10ms. Now, the camera may be operated at an acquisition frequency 2f_{b} i.e. 100Hz and therefore the maximal exposure time per frame in this example will be 10ms (/= 1/T). Hence in this example subsequent image frames will be acquired with and without the illumination by the light source. So, the difference image of subsequent frames contains only illumination by the light source and any effect of optical noise originating from the ambient illumination which is present in all the acquired frames gets cancelled in the difference image. By applying motion detection to the difference image, the effect of the noise on motion detection is eliminated.

In yet another exemplary embodiment the light exposure time of the camera frames may be chosen to avoid any light saturation of the camera sensor. The light exposure time may be chosen such that it is below a predetermined threshold by taking into consideration one or more parameters of the camera sensor and the frame rate of the camera.

In the exemplary embodiment described above, by modulating the amplitude of the light source with the camera acquisition frequency, subsequent image frames are acquired with and without the subject illumination. In yet another embodiment, the camera acquisition frequency may be chosen to match the modulation frequency of the light source. In an example, the camera may be operated in a phase locked mode with the modulation of the light source. Any phase locking algorithm may be used. By utilizing the phase locking mode, the camera can be precisely triggered to acquire image frames at twice the modulation frequency of the light source and to acquire subsequent image frames exactly with and without illumination from the light source.

The imaging device 110 comprises a movable subject table which is configured to support the subject during imaging procedures. Fig. 2 shows illustrative examples of the acquired camera image of a subject with different subject table positions. Fig. 2a and Fig. 2b are examples showing camera images of the subject with a shadow of a computed tomography (CT) gantry cast on the subject for a first and a second subject table position, respectively. Fig. 2a shows a camera image 200 with a dummy subject 204 supported on the subject table 206. The dummy subject 204 is for illustration purposes and any real subject or patient may be envisaged and placed on the subject table without deviating from the scope of the invention. The dummy subject 204 is herein referred to as a subject. An ambient light source (not shown in Fig. 2a) may cast a shadow on the subject 204. In this example CT gantry 208 casts a shadow on the subject 204 due to illumination by ambient light source. Such shadows often get unnoticed or undetected but can adversely affect the motion detection. An edge of the shadow is illustrated by numeral 210 in Fig. 2a. The subject table 206 is configured to move the subject during imaging procedures and also from an outside of the bore to the inside of the bore of the imaging device or the other way round. In either case, when subject table 206 moves subject 204 the shadow cast on the subject also moves along with. In Fig. 2b an edge of shadow 252 has moved to a second position due to movement of the subject table. Thus, as the subject table is moved the edge of the shadow moves across the subject.

Similarly, when the subject is inside the bore of the imaging device the computed tomography image acquisition is frequently done with a moving subject table. So, any shadow that is cast on the subject due to illumination inside the bore also moves with the subject and disturbs the motion detection algorithm. Any motion detection camera that acquires image frames of the subject to determine subject motion does not take into account the shadowing effect as described above.

Similarly, any spotlight of the ambient light source also moves across the subject during table motion and disturbs the motion detection algorithm.

The motion detection algorithm may assume that the pixels in the acquired image frames do not change color or brightness but only position over time. This assumption is incorrect here since the pixels in the respective camera frames may lose or gain brightness due to shadows or spotlights cast on the subject and movement of the subject inside and outside the shadow or spotlight zone with the subject table movement. Thus, motion detection is disturbed or perturbed leading to inaccurate motion data or missing motion data in the acquired frames due to the shadowing effect as described above. The drawbacks in motion detection as mentioned with reference to Fig. 2 are overcome by the system and method described with reference to Fig. 1.

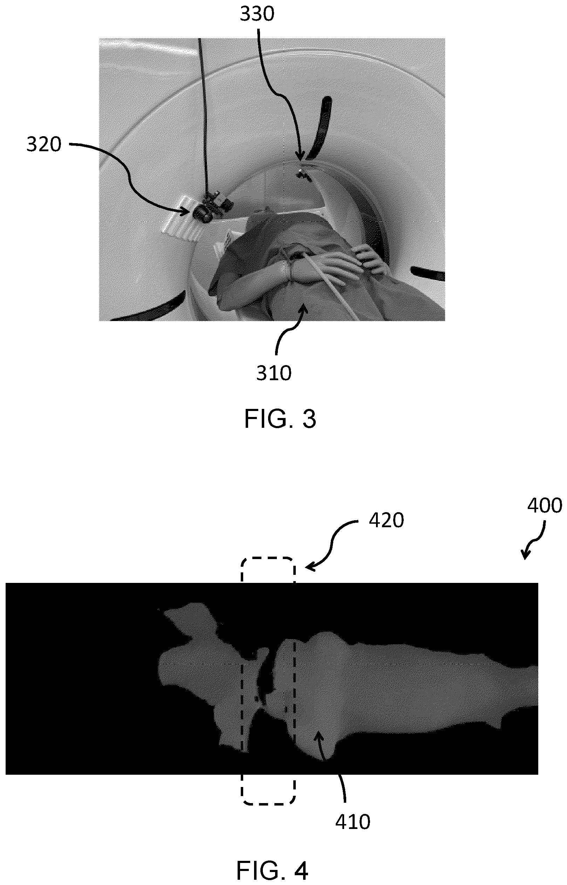

Reference is now made to Fig. 3, which shows an example of the subject on a CT table. Fig. 3 shows an embodiment where a camera 320 is positioned at a first end of the CT bore and a camera 330 at a second end of the CT bore. The cameras 320 and 330 may be used to monitor subject motion both before and during CT image acquisition. In another examples one or more cameras may be positioned at any other location of the CT bore. In an embodiment the bore may be illuminated by one or more light sources (not shown in Fig. 3). Thus, one or more cameras may monitor subject motion in presence of shadows or spotlights cast on the subject due to illumination by one or more light sources within and/or outside the bore of the imaging device.

Fig. 4 is an example image showing the effect of a shadow as in Figs 2a and 2b on motion monitoring. The image 400 in Fig. 4 represents motion data of the subject and 410 shows the acquired motion data of the subject. As mentioned above, the motion data of the subject may be acquired using one or more motion monitoring cameras. The motion data of the subject is acquired with a moving subject table as described above with reference to Fig. 2. As the subject with the subject table moves inside the CT bore any shadow cast on the subject may also move across the subject. This leads to the pixels in the acquired image frames losing color or brightness when the pixels move into the shadow zone thereby resulting in missing motion data in specific regions of the image as shown by numeral 420 in the Fig. 4.

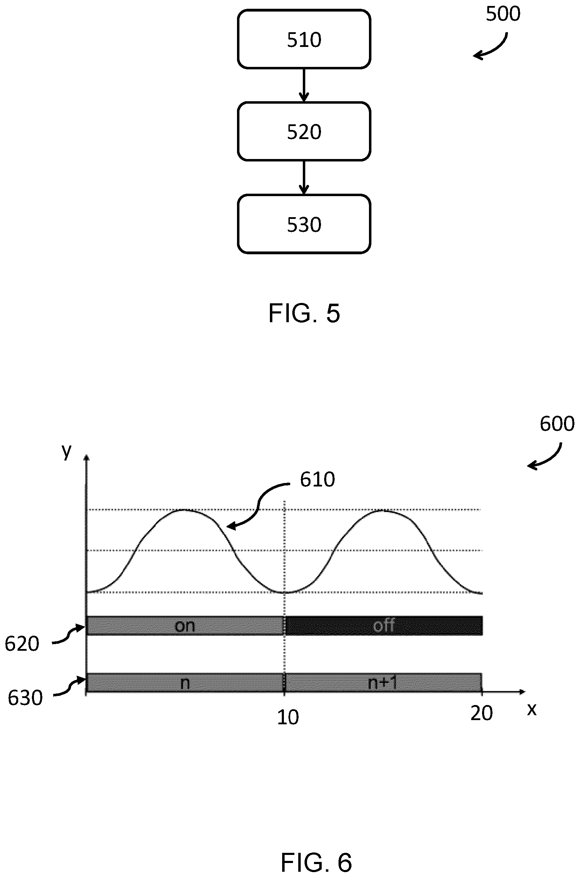

Fig. 5 is a flow chart showing a method for monitoring subject motion during a medical imaging procedure according to an embodiment of the invention.

The medical imaging procedure may comprise one or more further steps during or before the medical image acquisition using the imaging device. In an example, the medical imaging procedure may comprise a step of placing and/or positioning the subject on the subject table of the imaging device. In another example the medical imaging procedure may comprise a step of transitioning the subject from an outside to an inside of the bore of the imaging device. In yet another example, medical imaging procedure further comprises acquiring the medical image of the subject inside the bore of the imaging device.

At step 510, image frames of the subject are acquired by a camera. The image frames are acquired at a camera acquisition frequency.

The camera monitors subject motion by acquiring one or more image frames of the subject. The image frames of the subject may be acquired when the subject is in the field of view of the camera. The image frames may be acquired during the medical imaging procedure. The camera may acquire image frames when the subject is positioned on the table, or during transitioning the subject to an inside of the CT bore or during the CT image acquisition inside the bore. In this way the camera may continuously monitor the subject from the time the subject is at least in the field of view of the camera to the time when the CT system acquires images of the subject, and the imaging procedure is complete.

In further step 520 the subject is illuminated with light with a modulation frequency, the modulation frequency is synchronized with the camera acquisition frequency.

At step 530, subject motion is determined by applying motion detection algorithm to the acquired image frames. The motion detection algorithm is applied to a difference image of two acquired image frames.

The invention has advantages in that the difference image only contains light of the subject illumination, and any effect of ambient noise caused by light or shadows of the ambient illumination on the motion detection are removed.

In an embodiment of the invention, the one or more ambient light sources may have a pulsed light output. For example, most modern fluorescent and LED light sources are equipped with electronic drivers that modulate the light output of such light sources at very high frequencies far beyond the camera frame rate. Thus, the light output of such ambient light sources may be pulsed and will switch on and off multiple times within one camera frame acquisition. Such ambient light sources may therefore not disturb the motion detection. However, incandescent light bulbs or fluorescent light sources without such electronic drivers may be directly driven by AC mains power supply of 50 Hz (or 60 Hz). Such variation of light output although may not be perceived by human eye but can disturb the motion estimation. In this embodiment, the invention mitigates the effect of such variation of ambient light output by synchronizing the acquired camera frames with the subject lighting and also the ambient light output.

Fig. 6 illustrates the synchronization of the acquired camera frames with the subject lighting and the ambient illumination graphically. In the graphical illustration 600 of Fig. 6, the pulsed light output of ambient light is shown by curve 610. As illustrated by numeral 630 in the graph, within one camera frame acquired between 0 to 10 millisecond (x- axis of the graph) the ambient light output 610 changes over time but the camera detects only a mean ambient light output which is equal for any two consecutive camera frames n and n+1. The subject is illuminated by light source with a temporally modulated frequency illustrated by numeral 620 which is synchronized with the acquired frames 630 of the camera. Therefore, since each camera frame detects the mean ambient light output, the difference of the camera frames n and n+1 contains only subject lighting and no ambient lighting. The effect of ambient light gets cancelled and the impact of any shadows or spotlights due to the ambient lighting on motion detection is mitigated. In this way, the frequency of the light source and/or the camera acquisition frequency may be synchronized with the frequency of the ambient light source which has a pulsed light output.

In a further embodiment by selecting a high modulation frequency of the light source and a high acquisition frequency of the camera the effect of the subject table movement between two consecutive acquired camera frames is minimized. For example, for a table speed of 30mm/sec and a modulation frequency of the light source at f_{b}= 50 Hz, the subject moves by only 0.3 mm between the two acquired frames. This movement will be small to adversely affect any motion detection.

Any of the method steps disclosed herein may be recorded in the form of a computer program comprising instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid-state memory, magnetic tape, a removable computer diskette, a random-access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A medical imaging system comprising:
- an imaging device configured to acquire a medical image of a subject;
- a camera configured to acquire image frames of the subject, wherein the image frames are acquired at a camera acquisition frequency;
- a light source configured to illuminate the subject with a modulated frequency, wherein the modulation frequency is synchronized with the camera acquisition frequency;
- a motion detection unit configured to determine a subject motion by applying a motion detection algorithm to the acquired image frames, wherein the motion detection algorithm is applied to a difference image of two acquired image frames.

2. The medical imaging system according to claim 1, wherein the frequency of the light source is modulated by switching the light source on and off.

3. The medical imaging system according to claim 1 or 2, wherein the camera is further configured to operate in a phase locked mode with the illumination of the light source.

4. The medical imaging system according to any of claims 1-3, wherein the camera acquires subsequent image frames with and without the subject illumination.

5. The medical imaging system according to any of claims 1-4, wherein the light source is further configured to illuminate the subject with an intensity that varies in at least one spatial direction.

6. The medical imaging system according to any of claim 1-5, wherein the imaging device is further configured to receive ambient light illumination from at least one ambient light source.

7. The medical imaging system according to claim 6, wherein at least a frequency of the light source and the camera acquisition frequency are configured to be synchronized with a frequency of the ambient light source.

8. The medical imaging system according to claim 6 or 7 wherein the camera is further configured to detect a temporal mean ambient light level, wherein the temporal mean ambient light level is substantially equal for any two subsequent image frames acquired by the camera.

9. The medical imaging system according to any of claims 1-8, wherein the imaging device comprises a movable subject table which is configured to support the subject during the imaging procedure.

10. The medical imaging system according to any of claims 1-9, wherein the motion detection unit and the camera are built as a single unit.

11. The medical imaging system according to any of claims 1-10, wherein the imaging device is a computed tomography imaging system or a magnetic resonance imaging system.

12. The medical imaging system according to any of claims 1-11, wherein light exposure time of the acquired camera frames is below a predetermined threshold.

13. A system for monitoring motion of a subject during a medical imaging procedure, the system comprising:
- a camera configured to acquire image frames of the subject, wherein the frames are acquired at a camera acquisition frequency;
- a light source configured to illuminate the subject with a modulated frequency, wherein the modulation frequency is synchronized with the camera acquisition frequency;
- a motion detection unit configured to determine a subject motion by applying a motion detection algorithm to the acquired image frames, wherein the motion detection algorithm is applied to a difference image of two acquired image frames.

14. A method for monitoring motion of a subject during a medical imaging procedure, the method comprising:
- acquiring image frames of the subject by a camera, wherein the frames are acquired at a camera acquisition frequency;
- illuminating the subject with light with a modulated frequency, wherein the modulation frequency is synchronized with the camera acquisition frequency;
- determining the subject motion by applying a motion detection algorithm to the acquired image frames, wherein the motion detection algorithm is applied to a difference image of two acquired image frames.

15. A computer program product comprising instructions for causing a processor to carry out the method according to claim 14, when the computer program is executed.
